(19) Europäisches Patentamt
European Patent Office
Office européen des brevets

(11) Veröffentlichungsnummer: **0 158 267**
**A2**

(12) # EUROPÄISCHE PATENTANMELDUNG

(21) Anmeldenummer: **85103973.5**

(22) Anmeldetag: **02.04.85**

(51) Int. Cl.[4]: **A 61 K 31/55**
C 07 D 243/26

(30) Priorität: **11.04.84 DE 3413592**

(43) Veröffentlichungstag der Anmeldung:
**16.10.85 Patentblatt 85/42**

(84) Benannte Vertragsstaaten:
**AT BE CH DE FR GB IT LI NL SE**

(71) Anmelder: **Dolorgiet Beteiligungs-GmbH**
**Otto-von-Guericke-Strasse 1**
**D-5205 St. Augustin 3(DE)**

(72) Erfinder: **Posselt, Klaus, Dr.**
**Rodderbergstrasse 62**
**D-5300 Bonn 2(DE)**

(72) Erfinder: **Wagener, Hans Heinrich, Prof. Dr. med.**
**Breslauerstrasse 76**
**D-5309 Meckenheim(DE)**

(72) Erfinder: **Gruber, Klaus, Dr.**
**Münchenerstrasse 19**
**D-8024 Oberhaching(DE)**

(74) Vertreter: **Werner, Hans-Karsten, Dr. et al,**
**Deichmannhaus am Hauptbahnhof**
**D-5000 Köln 1(DE)**

(54) **Arzneimittel, enthaltend 5-(2-Fluorphenyl)-1,3-dihydro-3-hydroxy-7-nitro- oder 5-(2-Fluorphenyl)-1,3-dihydro-3-hydroxy-1-methyl-7-nitro-2H-1,4-benzodiazepin-2-on und Verfahren zur deren Herstellung.**

(57) 5-(2-Fluorphenyl)-1,3-dihydro-3-hydroxy-1-methyl-7-nitro- und 5-(2-Fluorphenyl)-1,3-dihydro-3-hydroxy-7-nitro-2H-1,4-benzodiazepin-2-on sind unbedeutende Metabolite des Flunitrazepams, die selbst ausgezeichnete pharmakologische Wirksamkeit aufweisen. Außer der Verwendung zur Herstellung von Arzneimitteln wird die Herstellung dieser Substanzen beschrieben.

EP 0 158 267 A2

Die Verbindung 5-(2-Fluorphenyl)-1,3-dihydro- 1-methyl-7-nitro-2H-1,4-benzodiazepin-2-on (genannt Flunitrazepam) ist aus der DE-PS 14 45 872 bekannt und wird in Arzneimitteln zur Behandlung von Schlafstörungen verwendet.

Bei Untersuchungen zur Aufklärung des Metabolismus von Flunitrazepam wurde auch die Bildung von 3-Hydroxyderivaten postuliert (J.A.F. de Silva et al. J. pharm. Sci. 63, 1974, 520-527; J.P. Cano et al. Arzneim.-Forsch./-Drug Res. 27, 1977, 338-342), da zugehörige Glukuronide identifiziert werden konnten (G. Wendt, Editiones Roche, Basel 1976, S. 27; eds. W. Hügin, G. Hossli, M. Gemperle). Diese 3-Hydroxyderivate spielen im Metabolismus von Flunitrazepam beim Menschen nur eine untergeordnete Rolle (U. Klotz et al. Progr. Pharmacol. 3, 1980, No. 3, S. 41). Angaben zur pharmakologischen Wirksamkeit und Herstellung der 3-Hydroxyderivate fehlen.

Aus den DE-OS 14 45 873, DE-OS 15 45 957 und DE-OS 16 95 163 sind 3-Hydroxy-7-nitro-benzodiazepine an sich bekannt. Die 5-(2-Fluor-phenyl)-derivate sind hier jedoch speziell nicht beschrieben. Auch fehlen jegliche Angaben über die pharmakologische Wirksamkeit dieser Substanzen.

In der DE-OS 33 14 893 ist ein verbessertes Verfahren zur Herstellung von 3-Hydroxy-benzodiazepin-2-onen durch Hydrolyse der entsprechenden Acyloxyverbindungen beschrieben, 5-(2-Fluorphenyl)-3-hydroxy-7-nitro-benzodiazepin-2-one sind jedoch nicht erwähnt. Die CH-PS 408 029 beschreibt ein Verfahren zur Nitrierung von Benzodiazepinen, die in 3-Stellung keinen Hydroxylrest enthalten.

Die Substanzen 5-(2-Fluorphenyl)-1,3-dihydro-3-hydroxy-7-nitro- und 5-(2-Fluorphenyl)-1,3-dihydro-3-hydroxy-1-methyl-7-nitro-2H-1,4-benzodiazepin-2-on sind somit bisher nur als unbedeutende Metabolite des Flunitrazepams beschrieben worden. Synthesen für diese Substanzen sind bisher nicht bekannt geworden.

Es wurde jetzt gefunden, daß diese Metabolite gute pharmakologische Wirksamkeit und günstige toxische Eigenschaften aufweisen und daher ausgezeichnet als Wirkstoffe von Arzneimitteln verwendet werden können.

Beispielsweise zeigte das 5-(2-Fluorphenyl)-1,3-dihydro-3-hydroxy-1-methyl-7-nitro-2H-1,4-benzodiazepin-2-on (DP 327) gegenüber Flunitrazepam im Kampftest eine etwa doppelt so starke tranquillisierende Wirkung. Hinsichtlich der schlafverlängernden Wirkung erwiesen sich beide Verbindungen zwar als gleichwertig, bei der toxikologischen Untersuchung stellte sich jedoch eine deutliche Überlegenheit der erfindungsgemäßen Verbindung heraus. DP 327 erwies sich als sehr wenig toxisch. Bis zur höchstmöglichen applizierbaren Dosis von 8687 mg/kg traten nur vereinzelt Todesfälle auf. Bei der Beobachtung des Zustandes der überlebenden Tiere konnten Vorteile von DP 327 gegenüber Flunitrazepam dahingehend registriert werden, daß die Tiere bis zu einer wesentlich höheren Dosis von DP 327 ohne Befund waren und darüber hinaus nur eine leichte Sedierung und Stereotypie auftrat. Beim Flunitrazepam reichten die Befunde von starker Sedierung und Stereotypie bis zum Koma. Die in Beispiel 12 der DE-OS 14 45 873 beschriebene Verbindung, die sich von der erfindungsgemäßen Substanz DP 327 nur dadurch unterscheidet, daß sie in 2-Stellung des 5-ständigen Phenylrings unsubstituiert ist, erwies sich bei geringerer schlafverlängernder Wirkung

toxischer als DP 327. Außerdem ist DP 327 in der schlafverlängernden und tranquillisierenden Wirkung und in der Toxizität dem aus der DE-PS 16 45 904 bekannten Schlafmittel 7-Chlor-5-(2-chlorphenyl)-1,3-dihydro-3-hydroxy-1-methyl-2H-1,4-benzodiazepin-2-on (Lormetazepam) deutlich überlegen. Hinzu kommt noch, daß DP 327 eine rasche Schlafinduktion bewirkt, während sie bei Flunitrazepam langsam einsetzt und bei Lormetazepam fehlt.

Auch das 5-(2-Fluorphenyl)-1,3-dihydro-3-hydroxy-7-nitro-2H-1,4-benzodiazepin-2-on (DP 370) zeigte eine gute tranquillisierende (Kampftest) und eine gute schlafverlängerde Wirkung bei einer wesentlich geringeren Toxizität als die genannten Vergleichssubstanzen.

Gegenstand der vorliegenden Erfindung sind somit Arzneimittel enthaltend 5-(2-Fluorphenyl)-1,3-dihydro-3-hydroxy-1-methyl-7-nitro- oder 5-(2-Fluorphenyl)-1,3-dihydro-3-hydroxy-7-nitro-2H-1,4-benzodiazepin-2-on sowie ihre Verwendung zur Herstellung von Arzneimitteln.

Die erfindungsgemäßen Arzneimittel eignen sich als Mittel zur Behandlung von Schlafstörungen und als Beruhigungsmittel. Sie erfüllen weitgehend die heute an ein gutes Schlafmittel zu stellenden Forderungen, wie kurze Wirkungsdauer, keine Nachwirkungen und geringe Toxizität.

Die Arzneimittel können oral appliziert oder injiziert werden und liegen als Tabletten, Dragees, Kapseln oder Lösungen vor.

Die Herstellung der Arzneimittel erfolgt unter Verwendung der bekannten, üblichen pharmazeutischen Hilfsstoffe sowie sonstiger üblicher Träger-, Verdünnungs-, Gleit- und Sprengmittel. Gegebenenfalls können Geschmacks- und Farbstoffe zugesetzt werden.

Als derartige Träger- und Hilfsstoffe kommen z.B. solche Stoffe in Frage, die in folgenden Literaturstellen als Hilfsstoffe für Pharmazie, Kosmetik und angrenzende Gebiete empfohlen bzw. angegeben sind: Ullmanns Encyclopädie der technischen Chemie, Band 4 (1953), Seiten 1 bis 39; H.P. Fiedler, Lexikon der Hilfsstoffe für Pharmazie, Kosmetik und angrenzende Gebiete, Editio Cantor, Aulendorf, 1981.

Beispiele hierfür sind Gelatine, Rohrzucker, Pektin, Stärke, Tylose, Talkum, Lycopodium, Kieselsäure, Milchzucker, Cellulosederivat, Stearate, Emulgatoren, pflanzliche Öle, Wasser, pharmazeutisch verträgliche ein- oder mehrwertige Alkohole und Polyglykole, wie Polyethylenglykole, sowie Derivate hiervon, Dimethylsulfoxid, Ester von aliphatischen gesättigten oder ungesättigten Fettsäuren mit ein- oder mehrwertigen Alkoholen, wie Glykolen, Diethylenglykol, Pentaerythrit, Sorbit, Mannit usw., die gegebenenfalls auch veräthert sein können, Benzylbenzoat, Dioxalane, Glyzerinformale, Glycolfurole, Dimethylacetamid, Lactamide, Lactate, Ethylcarbonate usw.

Zur Herstellung von Lösungen kommen beispielsweise Wasser oder physiologisch verträgliche organische Lösungsmittel in Frage, wie z.B. Ethanol, 1,2-Propylenglykol, Polyglykole und deren Derivate, Fettalkohole, Triglyceride, Partialester und Ketale des Glycerins, Paraffine und ähnliche, oder deren Mischungen.

Bei der Herstellung der Zubereitungen können bekannte und übliche Lösungsvermittler verwendet werden. Als Lösungsvermittler kommen beispielsweise in Frage: Polyoxyethylierte Fette, polyoxyethylierte Oleotriglyceride, linolisierte Oleotriglyceride.

Darüber hinaus ist der Zusatz von Konservierungsmitteln, Stabilisatoren, Puffersubstanzen, Geschmackskorrigenzien, Antioxidantien und Komplexbildnern (z.B. Ethylendiaminotetraessigsäure) und dergl. möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Als Antioxidantien kommen beispielsweise Natriummetabisulfit, Ascorbinsäure, Gallussäure, Gallussäurealkylester, Buthylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (z.B. Niederalkylester), Benzoesäure, Natriumbenzoat, Trichlorisobutylalkohol), Phenol, Cresol, Benzethoniumchlorid und Formalinderivate in Betracht.

Die pharmakologische und galenische Handhabung der erfindungsgemäßen Kombinationen erfolgt nach den üblichen Standardmethoden, wie z.B. in Ullmanns Encyclopädie der technischen Chemie, Band 18, Seiten 151 bis 161, Weinheim 1979, beschrieben sind.

Die Einzeldosis an 5-(2-Fluorphenyl)-1,3-dihydro-3-hydroxy-1-methyl-7-nitro- oder 5-(2-Fluorphenyl)-1,3-dihydro-3-hydroxy-7-nitro-2H-1,4-benzodiazepin-2-on kann beispielsweise zwischen 0,5 und 10 mg liegen, die in Abhängigkeit von der Schwere der Krankheit und dem Alter des Patienten ein- bis dreimal täglich verabreicht werden kann.

Die Herstellung von 5-(2-Fluorphenyl)-1,3-dihydro-3-hydroxy-1-methyl-7-nitro-2H-1,4-benzodiazepin-2-on erfolgt beispielsweise, indem man Ester der allgemeinen Formel

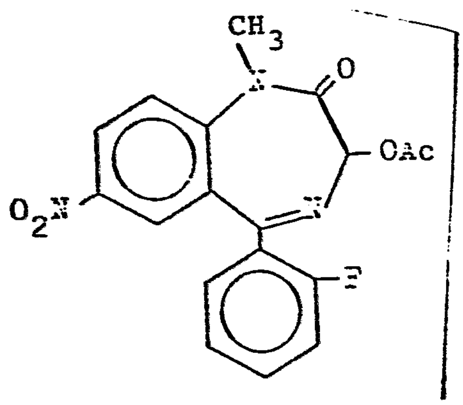

in der Ac ein Acylrest mit 2 bis 5 C-Atomen ist, in an sich bekannter Weise alkalisch oder sauer hydrolisiert.

Die alkalische Hydrolyse wird mit Ammoniak, Mono-, Di- oder Trialkylaminen, deren Alkylreste 1 bis 4 C-Atome enthalten, oder einem Alkalicyanid, oder Alkalihydroxid oder Alkalialkoxid, die saure Hydrolyse mit einer verdünnten Säure, vorzugsweise mit Salzsäure oder Schwefelsäure durchgeführt. Als Lösungsmittel kommen bei der alkalischen Hydrolyse Alkohol, vorzugsweise Methanol oder Ethanol, bei der sauren Tetrahydrofuran, Dioxan, Aceton oder Essigsäure in Frage. Die Hydrolyse wird bei

niederen Temperaturen im Bereich von 0 bis 50°C durchgeführt. Dabei hat sich gezeigt, daß, um befriedigende Ausbeuten zu erhalten, im alkalischen Medium eine Temperatur von 15 bis 25°C und bei der Verwendung von Schwefelsäure eine Temperatur von 0 bis 25°C einzuhalten ist und bei Verwendung von Salzsäure 50°C nicht überschritten werden dürfen.

Nach einer weiteren Variante kann 5-(2-Fluorphenyl)-1,3-dihydro-3-hydroxy-1-methyl-7-nitro-2H-1,4-benzodiazepin-2-on hergestellt werden durch Nitrieren einer Substanz der allgemeinen Formel

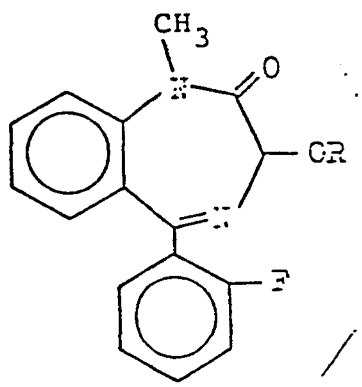

in der R Wasserstoff oder eine Acylgruppe mit 2 bis 5 C-Atomen ist.

Die Nitrierung wird mit Salpetersäure, einem Alkalinitrat oder Ammoniumnitrat bei Temperaturen zwischen 0 und 40°C in einem Schwefelsäure-Essigsäure-Gemisch, vorzugsweise im Verhältnis 2:1 bis 1:3 (v/v), durchgeführt.

Die Ausgangsverbindungen können in an sich bekannter Weise durch Umsetzen der N(4)-oxide mit einem Anhydrid der $C_2$- bis $C_5$-Carbonsäuren bei Temperaturen zwischen 80 und 120°C hergestellt werden.

Die N(4)-oxide erhält man durch Oxidation von Verbindungen der Formel

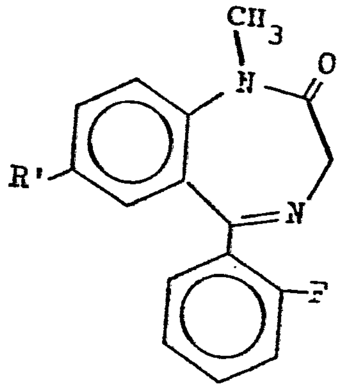

mit Wasserstoffperoxid oder einer Peroxicarbonsäure, vorzugsweise m-Chlorperoxibenzoesäure in einem inerten Lösungsmittel, vorzugsweise einem chlorierten Kohlenwasserstoff oder Essigsäure, bei Temperaturen zwischen 20 und 100°C.

Außerdem können die Ausgangsverbindungen durch Umsetzung mit Blei(IV)acetat in Gegenwart von Jod oder Alkalijodid in Essigsäure bei Temperaturen von 80 bis 120°C hergestellt werden.

Die Herstellung von 5-(2-Fluorphenyl)-1,3-dihydro-3-hydroxy-7-nitro-2H-1,4-benzodiazepin-2-on erfolgt dadurch, daß man Ester der allgemeinen Formel

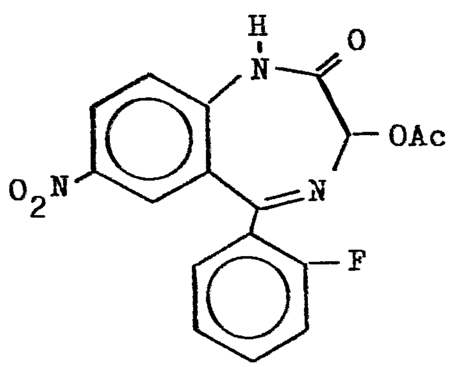

in der Ac ein Acylrest mit 2 bis 5 C-Atomen ist, in an sich bekannter Weise sauer hydrolysiert.

Die saure Hydrolyse wird mit einer verdünnten Säure, vorzugsweise mit Salzsäure oder Schwefelsäure durchgeführt. Als Lösungsmittel kommt vorzugsweise Essigsäure in Frage. Die Hydrolyse wird bei niederen Temperaturen im Bereich von 0 bis 30°C durchgeführt.

Ein weiterer Gegenstand der Erfindung ist somit die Herstellung von 5-(2-Fluorphenyl)-1,3-dihydro-3-hydroxy-1-methyl-7-nitro- und 5-(2-Fluorphenyl)-1,3-dihydro-3-hydroxy-7-nitro-2H-1,4-benzodiazepin-2-on, zumal diese zum Teil vom Stand der Technik erheblich abweichende Maßnahmen erfordert. 5-(2-Fluorphenyl)-1,3-dihydro-3-hydroxy-1-methyl-7-nitro-2H-1,4-benzodiazepin-2-on (DP 327) und 5-(2-Fluorphenyl)-1,3-dihydro-3-hydroxy-7-nitro-2H-1,4-benzodiazepin-2-on (DP 370) wurden wie folgt pharmakologisch untersucht:

(1) Verlängerung der Schlafzeit

Die Wirkung auf die Barbiturat-Narkose wurde in der Versuchsanordnung von J.M. Fujimoto et al. (J. Pharmacol. Exp. Ther. 129, 1960, 139) nachgewiesen. Gruppen von jeweils 10 männlichen Mäusen wurden die Testsubstanzen als Suspension in wässriger Carboxymethylcellulose mittels einer Stahlsonde auf oralem Weg direkt in den Magen verabreicht. 30 Minuten später wurde den Tieren 80 mg/kg Hexobarbital in eine Schwanzvene injiziert. Es wurde die Dauer des Schlafs gemessen und aus den Schlafzeiten der einzelnen Tiere der Kontroll- und Dosisgruppen die jeweiligen Mittelwerte berechnet. Die Verlängerung der Schlafzeit wurde in Prozent gegenüber der Kontrollgruppe berechnet.

(2) Kampftest

Die Hemmwirkung gegenüber strominduzierter Kampfhandlungen bei Mäusen wurde in der Versuchsanordnung von R. Tedeschi et al. (J. Pharmacol. Exp. Ther. 125, 1959, 28) ermittelt. 10 Paare weiblicher Mäuse werden am Vortag des Versuches 3 Minuten elektrischen Stromstößen von 3,5 mA, 5 Hz und 5 msec ausgesetzt. Am Versuchstag werden die Testsubstanzen als Suspension in wässriger Carboxymethylcellulose mittels Magensonde peroral verabreicht. 30 Minuten nach der Verabreichung werden jeweils ein Paar in einem Testkäfig wieder den Stromstößen ausgesetzt und die innerhalb von 3 Minuten ausgelösten Kampfhandlungen gezählt. Die Zahl der Kampfhandlungen der jeweiligen Dosisgruppen werden addiert und in Prozent gegenüber der Kontrollgruppe berechnet. Der $ED_{50}$-Wert wurde mittels halblogarithmischer Regression aus den Dosis-Wirkungsbeziehungen bestimmt.

(3) Akute Toxizität

Für diesen Versuch wurden je 10 männliche und weibliche Mäuse verwendet. Die Testsubstanzen wurden als Suspension in wässriger Carboxymethylcellulose per Magensonde verabreicht. Die Beobachtungszeit betrug 7 Tage. Die Ergebnisse wurden nach der Methode von G.T. Litchfield und F.G. Wilcoxon (J. Pharmacol. Exp. Ther. 96, 1949, 99) errechnet. Die Ergebnisse sind in der nachfolgenden Tabelle zusammengestellt.

Tabelle

| | DP 327 | DP 370 | Flunitrazepam | Lormetazepam | DE-OS 14 45 873 Beispiel 12 |
|---|---|---|---|---|---|
| Verlängerung der Schlafzeit in % bei der Maus nach peroraler Gabe von 1mg/kg KG. | 156 | 118 | 179 | 78 | 99 |
| Kampftest $ED_{100}$ mg/kg KG. Maus oral | 0,115 | 0,310 | 0,310 | 0,512 | 0,097 |
| Akute Toxizität $LD_{50}$ mg/kg KG. Maus oral | >8687* | >10.000* | 2700 (2140-3405) | 6300 (5223-7598) | 4700 (4022-5491) |
| Zustand nach 24 h | bis 3600 mg/kg ohne Befund; 4824-8687 mg/kg leichte Sedierung. | bis 4640 mg/kg ohne Befund; ab 4640 mg/kg leichte Sedierung Sedierung, und Ataxie. | bis 1100 mg/kg ohne Befund; ab 1100 mg/kg starke Sedierung, Koma, Stereotypie. | 900-1240 mg/kg leichte Sedierung; ab 1700 mg/kg zunehmend starke Sedierung. | bis 3200 mg/kg ohne Befund; ab 3200 mg/kg starke Sedierung, Koma. |

* höchste technisch applizierbare Dosis

In den nachfolgenden Beispielen ist die Herstellung von 5-(Fluorphenyl)-1,3-dihydro-3-hydroxy-1-methyl-7-nitro- und 5-(2-Fluorphenyl)-1,3-dihydro-3-hydroxy-7-nitro-2H-1,4-benzodiazepin-2-on sowie deren Verwendung zur Herstellung von Arzneimitteln näher erläutert.

Beispiel 1

Man trägt 233 g 3-Acetoxy-5-(2-fluorphenyl)-1,3-dihydro-1-methyl-7-nitro-2H-1,4-benzodiazepin-2-on in eine Lösung von 32 g Ammoniak in 2500 ml Methanol ein, rührt 4 Stunden bei 30°C und läßt über Nacht bei Raumtemperatur stehen. Nach dem Absaugen, Waschen mit Methanol und Trocknen erhält man 198 g 5-(2-Fluorphenyl)-1,3-dihydro-3-hydroxy-1-methyl-7-nitro-2H-1,4-benzodiazepin-2-on, das man aus 1800 ml Acetonitril-Dimethylformamid-Gemisch (4:5 v/v) umkristallisiert. Schmp. 215°-216°C unter Zersetzung. Der Ausgangsstoff wird folgendermaßen hergestellt:

A. Man löst 239,8 g Jod in 5 l Essigsäure bei 105°C, gibt innerhalb 5 Minuten 429 g Blei(IV)acetat (85 prozentig) und danach 392,5 g 5-(2-Fluorphenyl)-1,3-dihydro- 1-methyl-7-nitro-2H-1,4-benzodiazepin-2-on unter Rühren zu und hält die Temperatur 1 Stunde bei 105°C. Danach wird auf Raumtemperatur abgekühlt, 5 l Methylenchlorid zugegeben, abgesaugt und das Filtrat im Vakuum zur Trockne abdestilliert. Man löst den Rückstand in 6,5 l Methylenchlorid, filtriert und rührt das Filtrat zur Entfärbung mit Natriumthiosulfatlösung. Nach dem Trennen schüttelt man die wässrige Phase dreimal mit Methylenchlorid aus, vereinigt die organischen Phasen, wäscht mit Natriumbicarbonatlösung, trocknet über Natriumsulfat, behandelt mit Aktivkohle und destilliert das Lösungsmittel im Vakuum ab. Man erhält 241 g 3-Acetoxy-5-(2-fluorphenyl)-1,3-dihydro-3-hydroxy-1-methyl-7-nitro-2H-1,4-benzodiazepin-2-on. Schmp. 217-218°C. Umkristallisieren einer Probe aus Essigsäure ändert den Schmelzpunkt nicht.

B. Eine Lösung von 45 g 5-(2-Fluorphenyl)-1,3-dihydro-7-nitro-2H-1,4-benzodiazepin-2-on-4-oxid in 150 ml trockenem Dimethylformamid wird mit 7 g Natriumhydrid (50 prozentige Suspension in Öl) behandelt. Nachdem man 1 Stunde bei 70°C gerührt hat, kühlt man die Suspension und tropft 30 g Methyljodid zu. Danach wird 2 Stunden bei 50°C gerührt, abgekühlt, mit 300 ml Methylenchlorid verdünnt, mit Wasser ausgeschüttelt, getrocknet und das Lösungsmittel im Vakuum abdestilliert. Der Rückstand wird mit 500 ml siedendem Isopropanol behandelt. Man erhält 31 g 5-(2-Fluorphenyl)-1,3-dihydro-1-methyl-7-nitro-2H-1,4-benzodiazepin-2-on-4-oxid. Schmp. 193°C.
C. Eine Suspension von 30 g 5-(2-Fluorphenyl)-1,3-dihydro-1-methyl-7-nitro-2H-1,4-benzodiazepin-2-on-4-oxid in 220 ml Acetanhydrid wird unter Rühren im siedenden Wasserbad erhitzt. Nach 4 Stunden hat sich das Ausgangsmaterial gelöst und das Reaktionsprodukt beginnt auszukristallisieren. Nach dem Abkühlen saugt man ab und wäscht mit n-Hexan nach. Man erhält 24 g 3-Acetoxy-5-(2-fluorphenyl)-1,3-dihydro-1-methyl-7-nitro-2H-1,4-benzodiazepin-2-on. Schmp. 217-218°C.

Beispiel 2

Man löst 3,7 g 3-Acetoxy-5-(2-fluorphenyl)-1,3-dihydro-1-methyl-7-nitro-2H-1,4-benzodiazepin-2-on in 100 ml Tetrahydrofuran, setzt 10 ml 1n HCL zu, rührt 7 Stunden bei 40°C und läßt 2 Tage bei Raumtemperatur stehen. Dann neutralisiert man mit konzentriertem Ammoniak, trennt das Wasser ab, destilliert das Tetrahydrofuran im Vakuum ab, behandelt den Rückstand mit 50 ml siedendem Isopropanol und kristallisiert aus 60 ml Acetonitril um. Man erhält 1,5 g 5-(2-Fluorphenyl)-1,3-dihydro-3-hydroxy-1-methyl-7-nitro-2H-1,4-benzodiazepin-2-on. Schmp. 215-216°C.

Beispiel 3

Man versetzt eine Suspension von 3,7 g 3-Acetoxy-5-(2-fluorphenyl)-1,3-dihydro-1-methyl-7-nitro-2H-1,4-benzodiazepin-2-on in 80 ml Aceton mit 8 ml 1n HCL und rührt bei 40°C, wobei nach ca. 90 Minuten die Substanz in Lösung aufgegangen ist. Nach 6 Stunden kühlt man ab, neutralisiert mit konzentriertem Ammoniak und saugt ab. Nach Abdestillieren des Acetons vereinigt man beide feste Fraktionen, schüttelt mit Methylenchlorid-Wasser, trennt das Wasser ab, trocknet die organische Phase mit Natriumsulfat, destilliert ab und kristallisiert aus 75 ml Acetonitril um. Man erhält 2 g 5-(2-Fluorphenyl)-1,3-dihydro-3-hydroxy-1-methyl-7-nitro-2H-1,4-benzodiazepin-2-on. Schmp. 216-217°C.

Beispiel 4

Man löst 1,85 g 3-Acetoxy-5-(2-fluorphenyl)-1-methyl-7-nitro-1,3-dihydro-2H-1,4-benzodiazepin-2-on bei 0°C in 25 ml konz. Schwefelsäure-Eisessig-Gemisch (1:1 v/v), rührt 3 Stunden bei dieser Temperatur und läßt dann über Nacht bei Raumtemperatur stehen. Anschließend gießt man die Mischung auf 200 g Eis, neutralisiert mit konz. Ammoiak, saugt den Feststoff ab und wäscht mit Wasser nach. Nach dem Trocknen erhält man 1,6 g 5-(2-Fluorphenyl)-1,3-dihydro-3-hydroxy-1-methyl-7-nitro-2H-1,4- benzodiazepin-2-on, das nach Umkristallisieren aus 60 ml Acetonitril bei 216-217°C schmilzt.
Der Ausgangsstoff wird folgendermaßen hergestellt:

Eine Mischung von 68,1 g 5-(2-Fluorphenyl)-1-methyl-7-nitro-1,3-dihydro-2H-1,4-benzodiazepin-2-on und 48,7 g m-Chlor-peroxibenzoesäure in 800 ml 1,2 Dichlorethan wird 3 Stunden unter Rückfluß erhitzt. Man filtriert von der unlöslichen m-Chlorbenzoesäure ab, verdünnt das Filtrat mit 800 ml Dichlormethan, schüttelt nacheinander mit jeweils 500 ml 10 prozentiger Salzsäure, 10 prozentiger Natronlauge und Wasser aus, trocknet über Natriumsulfat und destilliert das Lösungsmittel ab. Der ölige Rückstand wird mit 200 ml Aceton verrührt. Nach Stehen über Nacht kristallisieren 34,6 g 5-(2-Fluorphenyl)-1-methyl-7-nitro-1,3-dihydro-2H-1,4-benzodiazepin-2-on-4-oxid aus. Durch Einengen der Acetonlösung auf 50 ml und Zugabe von 50 ml Ether erhält man weitere 12,8 g Substanz. Schmp. 193°C. Eine Suspension von 50,9 g 5-(2-Fluorphenyl)-1-methyl-7-nitro-1,3-dihydro-2H-1,4-benzodiazepin-2-on-4-oxid in 370 ml Acetanhydrid wird unter Rühren im siedenden Wasserbad erhitzt. Nach 4 Stunden hat sich der Ausgangsstoff gelöst und das Reaktionsprodukt beginnt auszukristallisieren. Nach dem Abkühlen saugt man ab und wäscht mit n-Hexan nach. Man erhält 45,1 g 3-Acetoxy-5-(2-fluorphenyl)-1-methyl-7-nitro-1,3-dihydro-2H-1,4- benzodiazepin-2-on. Schmp. 217-218°C.

Beispiel 5

Zur Suspension von 1,85 g 3-Acetoxy-5-(2-fluorphenyl)-1-methyl-7-nitro-1,3-dihydro-2H-1,4-benzodiazepin-2-on in 26 ml Methanol tropft man unter Rühren bei Raumtemperatur eine Lösung von 292 mg Diethylamin in 10 ml Methanol. Man rührt 8 Stunden weiter, läßt über Nacht stehen, bringt die Mischung mit Essigsäure auf pH 5, saugt den Feststoff ab und wäscht mit Wasser neutral. Man erhält nach dem Trocknen 1,4 g 5-(2-Fluorphenyl)-1,3-dihydro-3-hydroxy-1-methyl-7-nitro-2H-1,4-benzodiazepin-2-on, das, aus 50 ml Acetonitril umkristallisiert, bei 216-217°C schmilzt.

Beispiel 6

Zur Suspension von 1,85 g 3-Acetoxy-5-(2-fluorphenyl)-1-methyl-7-nitro-1,3-dihydro-2H-1,4-benzodiazepin-2-on in 26 ml Methanol tropft man unter Rühren bei Raumtemperatur eine Lösung von 56 mg Kaliumhydroxid in 10 ml Methanol und rührt 2 Stunden weiter. Danach säuert man die Mischung mit Essigsäure bis pH 5 an, saugt den Feststoff ab, wäscht mit Wasser neutral und trocknet. Man erhält 1,4 g 5-(2-Fluorphenyl)-1,3-dihydro-3-hydroxy-1-methyl-7-nitro-2H-1,4-benzodiazepin-2-on, das aus 60 ml Acetonitril umkristallisiert, bei 216-217°C schmilzt.

Beispiel 7

Zur Suspension von 1,85 g 3-Acetoxy-5-(2-fluorphenyl)-1-methyl-7-nitro-1,3-dihydro-2H-1,4-benzodiazepin-2-on in 80 ml Methanol tropft man unter Rühren bei Raumtemperatur eine Lösung von 160 mg Kaliumcyanid in 10 ml Methanol. Nach 4 Stunden stellt man die Mischung mit Essigsäure auf pH 5 ein, saugt den Feststoff ab, wäscht mit Wasser neutral und trocknet. Man erhält 1,4 g 5-(2-Fluorphenyl)-1,3-dihydro-3-hydroxy-1-methyl-7-nitro-2H-1,4-benzodiazepin-2-on. Schmp. 216-217°C.

Beispiel 8

Zur Suspension von 1,85 g 3-Acetoxy-5-(2-fluorphenyl)-1-methyl-7-nitro-1,3-dihydro-2H-1,4-benzodiazepin-2-on in 26 ml Methanol tropft man unter Rühren bei Raumtemperatur 10 ml einer Natriummethylatlösung (hergestellt durch Lösen von 54 mg Natrium in 10 ml Methanol) und rührt 2 Stunden weiter. Danach säuert man die Mischung mit Essigsäure bis pH 5 an, saugt den Feststoff ab und wäscht mit Wasser neutral. Nach dem Trocknen erhält man 1,4 g 5-(2-Fluorphenyl)-1,3-dihydro-3-hydroxy-1-methyl-7-nitro-2H-1,4-benzodiazepin-2-on, das aus 60 ml Acetonitril umkristallisiert, bei 216-217°C schmilzt.

Beispiel 9

Man löst 1,63 g 3-Acetoxy-5-(2-fluorphenyl)-1-methyl-1,3-dihydro-2H-1,4-benzodiazepin-2-on bei 25°C in 10 ml konz. Schwefelsäure-Eisessig-Gemisch (1:1, v/v) und tropft unter Rühren bei 27°C 0,71 ml Salpetersäure-Schwefelsäure-Gemisch (hergestellt aus 2,95 ml Schwefelsäure d=1,84 und 3,18 ml Salpetersäure d=1,40) zu. Nach dreistündigem Rühren läßt man 48 Stunden bei 29°C stehen. Anschließend gießt man auf 200 g Eis, stellt mit konz. Ammoniak auf pH 6 ein, saugt ab und wäscht mit Wasser nach. 1,3 g trockene Substanz löst man unter Zusatz von Kohle mit 30 ml siedendem Acetonitril, filtriert und läßt bei Raumtemperatur über Nacht kristallisieren. Das Kristallisat wird in 8 ml siedendem Dimethylformamid-Acetonitril-Gemisch (3:2, v/v) gelöst und bei Raumtemperatur über Nacht stehen gelassen. Man erhält 0,5 g 5-(2-Fluorphenyl)-1,3-dihydro-3-hydroxy-1-methyl-7-nitro-2H-1,4-benzodiazepin-2-on mit Schmelzpunkt von 215-216°C. Der Mischschmelzpunkt mit einer nach Beispiel 1 hergestellten Probe ergibt keine Depression.

Beispiel 10

Man löst 1,42 g 5-(2-Fluorphenyl)-1,3-dihydro-3-hy-

droxy-1-methyl-7-nitro-2H-1,4-benzodiazepin-2-on bei 30°C in 10 ml konz. Schwefelsäure-Eisessig-Gemisch (1:2, v/v) und tropft unter Rühren eine Lösung von 0,51 g Kaliumnitrat in 3 ml konz. Schwefelsäure zu. Man hält 3 Stunden bei 35°C und läßt über Nacht bei Raumtemperatur stehen. Danach wird nochmals eine Lösung von 0,1 g Kaliumnitrat in 0,6 ml konz. Schwefelsäure zugegeben und 2 Stunden bei 35°C gehalten. Daraufhin wird wie in Beispiel 9 beschrieben aufgearbeitet. Man erhält 0,4 g 5-(2-Fluorphenyl)-1,3-dihydro-3-hydroxy-1-methyl-7-nitro-2H-1,4-benzodiazepin-2-on mit Schmelzpunkt von 215-216°C.

Beispiel 11

Man löst 1,8 g 3-Acetoxy-5-(2-Fluorphenyl)-7-nitro-1,3-dihydro-2H-1,4-benzodiazepin-2-on bei 0°C in 25 ml konz. Schwefelsäure-Eisessig-Gemisch (1:1 v/v), rührt 6 Stunden bei dieser Temperatur und läßt dann über Nacht bei Raumtemperatur stehen. Anschließend gießt man auf 200 g Eis, neutralisiert mit konz. Ammoniak, saugt den Feststoff ab und wäscht mit Wasser nach. Nach dem Trocknen erhält man 1,4 g 5-(Fluorphenyl)-3-hydroxy-7-nitro-1,3-dihydro-2H-1,4-benzodiazepin-2-on, das nach Umkristallisieren aus 30 ml Acetonitril bei 187 bis 188°C schmilzt. Der Ausgangsstoff wird folgendermaßen hergestellt:

Man löst 6 g Jod in 100 ml Eisessig, erhitzt auf 105°C, gibt innerhalb 5 Minuten 10,9 g Blei(IV)acetat (85 prozentig) und anschließend 9,5 g 5-(2-Fluorphenyl)-7-nitro-1,3-dihydro-2H-1,4-benzodiazepin-2-on unter Rühren zu und hält 1 Stunde bei 105°C. Nach Abkühlen auf Raumtemperatur gibt man 140 ml Methylenchlorid zu, rührt kurz, saugt ab und destilliert das Filtrat im Vakuum

zur Trockene ab. Man löst den Rückstand in 180 ml Methylenchlorid, filtriert und rührt das Filtrat bis zur Entfärbung mit Natriumthiosulfatlösung. Nach dem Trennen schüttlelt man die wässrige Phase 3 mal mit jeweils 100 ml Methylenchlorid aus, vereinigt die organischen Phasen, wäscht mit Natriumbicarbonatlösung, trocknet über Natriumsulfat, behandelt mit Aktivkohle und destilliert das Lösungsmittel im Vakuum ab. Man erhält 12,2 g 3-Acetoxy-5-(2-Fluorphenyl)-7-nitro-1,3-dihydro-2H-1,4-benzodiazepin-2-on. Schmp. 234-235°C. Umkristallisieren einer Probe aus Aceton/n-Hexan ändert den Schmelzpunkt nicht.

Beispiel 12

Tabletten

A. Die Bestandteile

| | |
|---|---|
| Wirkstoff gemäß Beispiel 1, fein gemahlen | 200 g |
| Hochdisperse Kieselsäure | 25 g |
| Natriumlaurylsulfat | 200 g |
| Alginsäure | 400 g |
| Mikrokristalline Cellulose | 7075 g |
| Magnesiumstearat | 100 g |

werden gut gemischt und anschließend direkt zu Tabletten verpreßt.

1 Tablette von 80 mg Gewicht enthält 2 mg Wirkstoff.

B. Die Bestandteile

| | |
|---|---|
| Wirkstoff gemäß Beispiel 11, fein gemahlen | 300 g |
| Hochdisperse Kieselsäure | 25 g |
| Natriumlaurylsulfat | 200 g |
| Alginsäure | 400 g |
| Mikrokristalline Cellulose | 6975 g |
| Magnesiumstearat | 100 g |

werden gut vermischt und anschließend direkt zu Tabletten verpreßt.
1 Tablette von 80 mg Gewicht enthält 3 mg Wirkstoff.

Beispiel 13

Überzogene Tabletten bzw. Dragées

Tabletten oder Dragéekerne, die entsprechend Beispiel 11 hergestellt werden, lassen sich in bekannter Weise (z.B. Ullmanns Encyklopädie der technischen Chemie, Band 18, Seite 158, Weinheim 1979) mit einem Film- oder einem Dragéeüberzug versehen. Als Filmbildner werden Cellulosederivate, Copolymerisate mit kationischem Charakter auf Basis von Dimethylaminoethylmethacrylat und Methacrylsäure bzw. Copolymerisate mit anionischem Charakter auf Basis von Methacrylsäure und Methacrylsäureestern eingesetzt. Für Dragéeüberzüge werden vorzugsweise Saccharose, Mannit oder Xylit verwendet.

Beispiel 14

Gelatine-Steckkapseln

Die Bestandteile

| | | |
|---|---|---|
| Wirkstoff gemäß Beispiel 1, fein gemahlen | 100 | g |
| Hochdisperse Kieselsäure | 25 | g |
| Natriumlaurylsulfat | 200 | g |
| Alginsäure | 400 | g |
| Mikrokristalline Cellulose | 7175 | g |
| Magnesiumstearat | 100 | g |

werden gut gemischt und anschließend in Einzelmengen von 80 mg in Gelatine-Steckkapseln der Größe 4 abgefüllt.
1 Kapsel enthält 1 mg Wirkstoff.

Beispiel 15

Ampullen

10 g Wirkstoff gemäß Beispiel 1 werden in einer Mischung aus 9 l Tetrahydrofurfurylalkoholpolyethylenglykolethylether, 100 ml Wasser für Injektionszwecke und 20 ml Essigsäure gelöst. Die Lösung wird mit Tetrahydrofurfurylalkoholpolyethylenglykolethylether auf 10 l aufgefüllt und filtriert. Nach Abfüllen der Lösung in Ampullen zu 2 ml wird in üblicher Weise sterilisiert.
1 Ampulle enthält 2 mg Wirkstoff in 2 ml Lösung.

## Patentansprüche

1. Arzneimittel enthaltend 5-(2-Fluorphenyl)-1,3-dihydro-3-hydroxy-1-methyl-7-nitro-2H-1,4-benzodiazepin-2-on.

2. Verwendung von 5-(2-Fluorphenyl)-1,3-dihydro-3-hydroxy-1-methyl-7-nitro-2H-1,4-benzodiazepin-2-on zur Herstellung von Arzneimitteln.

3. Verfahren zur Herstellung von 5-(2-Fluorphenyl)-1,3-dihydro-3-hydroxy-1-methyl-7-nitro-2H-1,4-benzodiazepin-2-on, dadurch gekennzeichnet, daß man entweder die entsprechenden 3-Acylderivate mit 2 bis 5 C-Atomen im Acylrest alkalisch oder sauer hydrolysiert oder die entsprechenden 3-Hydroxy- oder 3-Acyloxy-7-hydroverbindungen nitriert.

4. Arzneimittel enthaltend 5-(Fluorphenyl)-1,3-dihydro-3-hydroxy-7-nitro-2H-1,4-benzodiazepin-2-on.

5. Verwendung von 5-(Fluorphenyl)-1,3-dihydro-3-hydroxy-7-nitro-2H- 1,4-benzodiazepin-2-on zur Herstellung von Arzneimitteln.

6. Verfahren zur Herstellung von 5-(2-Fluorphenyl)-1,3-dihydro-3-hydroxy-7-nitro-2H-1,4-benzodiazepin-2-on, dadurch gekennzeichnet, daß man die entsprechenden 3-Acylderivate mit 2 bis 5 C-Atomen im Acylrest sauer hydrolysiert.